# EUROPÄISCHE PATENTANMELDUNG

(11) **EP 3 029 018 A1**
(43) Veröffentlichungstag der Anmeldung: **08.06.2016**
(21) Anmeldenummer: 14196650.7
(22) Anmeldetag: 05.12.2014
(51) Int. Cl.: C07C 7/09, C07C 11/06

(54) **Verfahren und Anlage zur Gewinnung von Propylen**

(71) Anmelder: Linde Aktiengesellschaft, 80331 München (DE)
(72) Erfinder: Hoefel, Dr. Torben, 81543 München (DE); Conradt, Joerg, 80639 München (DE); Pham Duc, Tuat, 82377 Penzberg (DE); Hesse, Andreas, 82515 Wolfratshausen (DE); Nold, Michael, 82515 Wolfratshausen (DE); Reyneke, Hendrik, 81479 München (DE); Wellenhofer, Anton, 82069 Hohenschäftlarn (DE); Stahl, Bernhard, 81379 München (DE); Bölt, Heinz, 82515 Wolfratshausen (DE); Holzapfel, Ina, 81479 München (DE)
(74) Vertreter: Frommberger, Moritz

(57) **Zusammenfassung**

Es wird ein Verfahren (100) zur Gewinnung von Propylen vorgeschlagen, bei dem ein Trenneinsatzstrom (G) bereitgestellt wird, der in einem Dehydrierungsschritt (101) aus Propan gebildetes Propylen sowie Propan, Kohlenwasserstoffe mit zwei Kohlenstoffatomen, Methan und Wasserstoff enthält, wobei aus Fluid des Trenneinsatzstroms (G) auf einem überatmosphärischen Druckniveau ein Propan und Propylen enthaltender und an Kohlenwasserstoffen mit zwei Kohlenstoffatomen, Methan und Wasserstoff armer erster Trennabstrom (S) gebildet wird. Es ist vorgesehen, dass aus Fluid des Trenneinsatzstroms (G) nach Abkühlen von einem ersten auf ein zweites Temperaturniveau ein erster flüssiger Kondensatstrom (c) und ein erster gasförmiger Reststrom (b) gebildet werden, aus Fluid des ersten gasförmigen Reststroms (b) nach weiterem Abkühlen auf ein drittes Temperaturniveau ein zweiter flüssiger Kondensatstrom (d) und ein zweiter gasförmiger Reststrom (e) gebildet werden, aus Fluid des ersten (c) und des zweiten flüssigen Kondensatstroms (d) der erste (S) und ein Methan und Kohlenwasserstoffe mit zwei Kohlenstoffatomen enthaltender und an Wasserstoff und Kohlenwasserstoffen mit mehr als drei Kohlenstoffatomen armer zweiter Trennabstrom (m) gebildet wird, aus Fluid des zweiten gasförmigen Reststroms (e) ein zumindest Propylen und Propan enthaltender und an Wasserstoff abgereicherter dritter Trennabstrom (x) gebildet wird, und der Trenneinsatzstrom (G) unter Verwendung von Fluid des dritten Trennabstroms (x) gebildet wird. Eine entsprechende Anlage ist ebenfalls Gegenstand der Erfindung.

## Beschreibung

Die Erfindung betrifft ein Verfahren und eine Anlage zur Gewinnung von Propylen gemäß den Oberbegriffen der unabhängigen Patentansprüche.

### Stand der Technik

Verfahren zur Dehydrierung von Alkanen, insbesondere von Propan zu Propylen, sind bekannt und beispielsweise im Artikel "Propene" in Ullmann's Encyclopedia of Industrial Chemistry, Onlineausgabe, 15. Juni 2000, DOI 10.1002/14356007.a22_211, Abschnitt 4.3., "Propane Deydrogenation", beschrieben.

Zur Dehydrierung von Propan zu Propylen werden beispielsweise Reaktoren verwendet, die mittels Brennern beheizte Brennkammern aufweisen, durch welche mehrere Reaktionsrohre geführt sind. Die Reaktionsrohre werden von außen durch die Brenner beheizt. Die Reaktionsrohre enthalten einen Katalysator auf einem geeigneten Träger. Geeignete Katalysatoren sind beispielsweise bei Bölt, H., "Dehydrierung von Propan/Butan", Linde Berichte aus Technik und Wissenschaft 66/1991, beschrieben. Durch die Reaktionsrohre wird das zu dehydrierende Propan zusammen mit Dampf, sogenanntem Prozessdampf, geführt. Zuvor erfolgt typischerweise eine Vorwärmung mittels Abwärme. Ein dem Reaktor bzw. entsprechenden Reaktionsrohren entnommenes Gasgemisch, nachfolgend auch als Produktstrom bezeichnet, wird einer geeigneten Produktaufbereitung zugeführt.

Die Dehydrierung von Propan zu Propylen ist bei der Wahl geeigneter Katalysatoren und Reaktionsbedingungen hochselektiv und kann bei über 90% liegen. Dennoch entstehen immer auch geringe Mengen an Nebenprodukten in Form von Wasserstoff, Methan sowie Kohlenwasserstoffen mit zwei und mehr als drei Kohlenstoffatomen. Ferner finden sich in einem entsprechenden Produktstrom immer auch größere Mengen an nicht zu Propylen umgesetztem Propan.

Wie auch unter Bezugnahme auf die beigefügten Figuren erläutert, gestaltet sich insbesondere die Abtrennung von Methan und von Kohlenwasserstoffen mit zwei Kohlenstoffatomen sowie Wasserstoff in Form einer sogenannten C2- oder C2minus-Fraktion (siehe unten) aus dem Produktstrom häufig als herausfordernd. Eine derartige Trennung muss tendenziell mit sehr geringen Temperaturen erfolgen, um zu vermeiden, dass größere Mengen an Kohlenwasserstoffen mit drei und mehr Kohlenstoffatomen, insbesondere das zur Dehydrierung zurückzuführende Propan und das als Zielprodukt auszuführende Propylen, in die C2- oder C2minus-Fraktion übergehen. Die Trennung ist besonders aufwendig, wenn im Verhältnis zu Methan und Kohlenwasserstoffen mit zwei Kohlenstoffatomen eine vergleichsweise hohe Wasserstoffkonzentration im Produktstrom vorliegt.

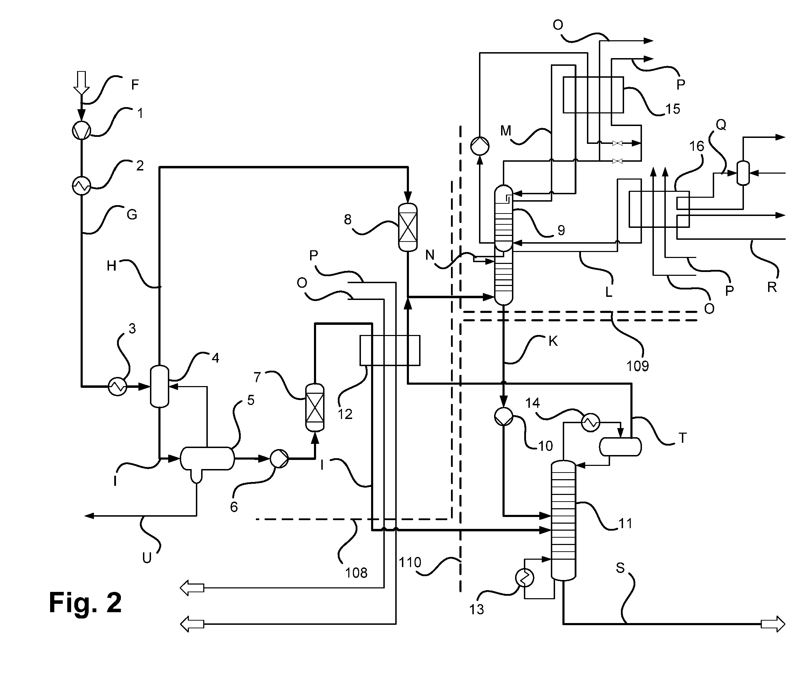

Ein Ansatz zur Lösung dieses Problems ist in der beigefügten Figur 2 gezeigt und unten unter Bezugnahme auf diese Figur erläutert. Dieser hat zum Ziel, nur konventionelle Kältekreisläufe mit nicht weniger als -40 °C (d.h. sogenannte C3-Kälte, also Kälte, die mittels Propan oder Propylen als Kältemittel geliefert werden kann) zu verwenden. Ferner sollen vorteilhafterweise keine Expansionsturbinen zum Einsatz kommen. Kälte auf einem Niveau von weniger als -40 °C kann hier bereitgestellt werden, indem aus dem Produktstrom kondensierte Kohlenwasserstoffe entspannt und zu einen Rohgasverdichtungsschritt zurückgeführt werden. Hierdurch wird ein integrierter Kältekreislauf geschaffen. In einer Anlage gemäß Figur 2 werden durch eine vertretbare Menge eines Recyclestroms jedoch nur Temperaturen von bis zu ca. -75 °C erzeugt, so dass ein Verlust von ca. 1% von Kohlenwasserstoffen mit drei Kohlenstoffatomen in die C2- oder C2minus-Fraktion unvermeidlich ist.

Die Erfindung stellt sich vor diesem Hintergrund die Aufgabe, herkömmliche Verfahren und Anlagen zur Gewinnung von Propylen zu verbessern.

### Offenbarung der Erfindung

Diese Aufgabe wird durch ein Verfahren und eine Anlage zur Gewinnung von Propylen mit den Merkmalen der unabhängigen Patentansprüche gelöst. Bevorzugte Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ±1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Flüssige und gasförmige Ströme können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält. Ist hier beispielsweise vereinfacht von "Propan" die Rede, sei darunter ein Strom verstanden, der reich an Propan ist, jedoch nicht ausschließlich aus Propan bestehen muss. Ist hier und im Folgenden davon die Rede, dass "aus Fluid" eines Stroms ein weiterer Strom gebildet wird, kann hierunter sowohl fallen, dass der weitere Strom aus der gesamten Menge des Fluids des jeweiligen Ausgangsstroms gebildet wird, aber auch, dass der weitere Strom nur aus einem Teil hiervon gebildet wird.

Vorliegend können zur Trennung von Kohlenwasserstoffströmen insbesondere die Destillationssäulen und Absorptionskolonnen zum Einsatz kommen. Zur Auslegung und Ausgestaltung entsprechender Vorrichtungen sei auf einschlägige Lehrbücher verwiesen (siehe beispielsweise K. Sattler: Thermische Trennverfahren. Grundlagen, Auslegung, Apparate. Weinheim: Wiley-VCH, 3. Auflage 2001). Destillationssäulen und Absorptionskolonnen werden nachfolgend auch unter dem Begriff "Trennsäulen" zusammengefasst. Eine im Rahmen der vorliegenden Erfindung verwendete Trennsäule wird bei tiefkalten Temperaturen betrieben und ist zur Tieftemperatur-Gaszerlegung eingerichtet. Einer Trennsäule ist typischerweise immer zumindest eine flüssige Fraktion ("Sumpfprodukt") und eine gasförmige Fraktion ("Kopfprodukt") in einem oberen ("Kopf") bzw. unteren Bereich ("Sumpf") entnehmbar.

Gängige Verfahren zur Trennung von Produktströmen aus Verfahren zur Herstellung von Kohlenwasserstoffen umfassen die Bildung einer Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (und konventionsgemäß unter Umständen auch Wasserstoff, dann auch "C1minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für die "C5-Fraktion" und die höheren Fraktionen. Mehrere solcher Fraktionen können zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

### Vorteile der Erfindung

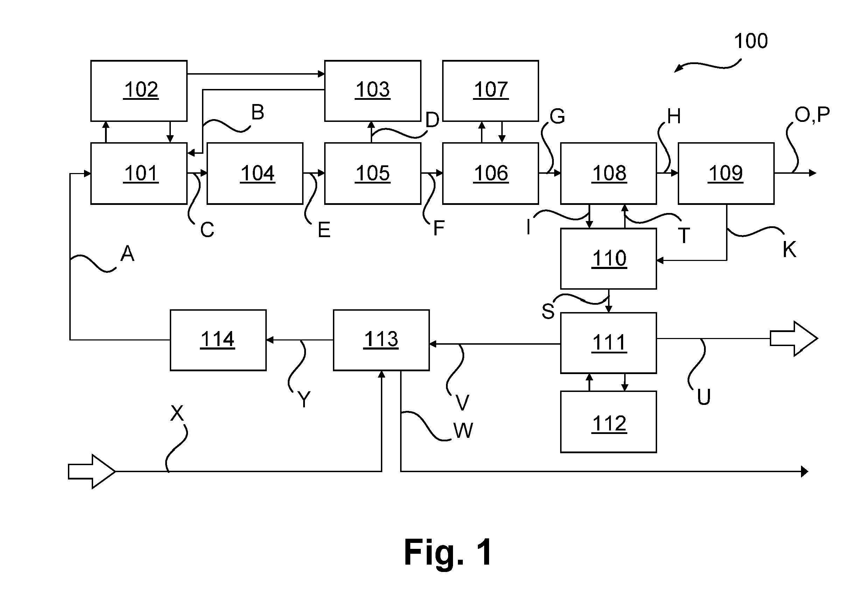

Die vorliegende Erfindung geht von einem Verfahren zur Gewinnung von Propylen aus, bei dem ein Trenneinsatzstrom bereitgestellt wird, der in einem Dehydrierungsschritt aus Propan gebildetes Propylen sowie Propan, Kohlenwasserstoffe mit zwei Kohlenstoffatomen, Methan und Wasserstoff enthält. Ein entsprechender Trenneinsatzstrom kann aus einem sogenannten Rohgasstrom eines entsprechenden Dehydrierungsschritts gebildet werden, indem, wie auch unter Bezugnahme auf die Figur 1 erläutert, aus einem derartigen Rohgasstrom bestimmte Komponenten entfernt werden und der Rohgasstrom gekühlt und verdichtet wird. Ein entsprechender Trenneinsatzstrom kann neben den genannten Komponenten auch insbesondere noch Kohlenwasserstoffe mit mehr als drei Kohlenstoffatomen enthalten. Methylacetylen kann ebenfalls als Kohlenwasserstoff mit drei Kohlenstoffatomen enthalten sein.

Wie grundsätzlich bekannt, wird aus Fluid des Trenneinsatzstroms auf einem überatmosphärischen Druckniveau ein Propan und Propylen enthaltender und an Kohlenwasserstoffen mit zwei Kohlenstoffatomen, Methan und Wasserstoff armer erster Trennabstrom gebildet. Aus dem Trenneinsatzstrom wird also ein C3- oder C3plus-Strom erzeugt, der Propan, das in den Dehydrierungsschritt zurückzuführen ist, Propylen, das aus der Anlage auszuleiten ist, und gegebenenfalls Methylacetylen, vorteilhafterweise aber keine leichteren Komponenten enthält. Sind in dem Trenneinsatzstrom Kohlenwasserstoffe mit vier und mehr Kohlenstoffatomen enthalten, gehen diese typischerweise ebenfalls in den ersten Trennabstrom über. Zur Bedeutung des Begriffs "arm" sei auf die obigen Erläuterungen verwiesen. Insbesondere enthält der erste Trennabstrom Kohlenwasserstoffe mit zwei Kohlenstoffatomen, Methan und Wasserstoff in einem Gehalt von weniger als 5%.

Die Erfindung sieht dabei vor, aus Fluid des Trenneinsatzstroms nach Abkühlen von einem ersten auf ein zweites Temperaturniveau einen ersten flüssigen Kondensatstrom und einen ersten gasförmigen Reststrom zu bilden. Ferner wird aus Fluid des ersten gasförmigen Reststroms nach weiterem Abkühlen auf ein drittes Temperaturniveau ein zweiter flüssiger Kondensatstrom und ein zweiter gasförmiger Reststrom gebildet. Es erfolgt also bis zu diesem Punkt eine zweistufige Kondensation auf unterschiedlichen Temperaturniveaus, auf denen jeweils Kondensate in Form der ersten und zweiten flüssigen Kondensatströme anfallen und gasförmige Restströme verbleiben. Hierdurch können die jeweils erhaltenen Kondensate an schwereren Komponenten an- und an leichteren Komponenten abgereichert werden, so dass sich die Trennung im Folgenden vereinfacht. Zur Bildung der Kondensatströme und der gasförmigen Restströme sind dabei vorteilhafterweise geeignete Wärmetauscher vorgesehen, denen Abscheidebehälter nachgeschaltet sind, in deren Sumpf jeweils die flüssigen Kondensatströme und an deren Kopf jeweils die gasförmigen Restströme anfallen.

Aus Fluid des ersten und des zweiten flüssigen Kondensatstroms wird erfindungsgemäß, insbesondere destillativ, der zuvor erläuterte erste Trennabstrom sowie ein Methan und Kohlenwasserstoffe mit zwei Kohlenstoffatomen enthaltender und an Wasserstoff und Kohlenwasserstoffen mit mehr als drei Kohlenstoffatomen armer zweiter Trennabstrom gebildet. Dadurch, dass ein entsprechender zweiter Trennabstrom aus dem ersten und zweiten flüssigen Kondensatstrom gebildet wird und nicht direkt aus dem Trenneinsatzstrom, ist dieser ausgesprochen wasserstoffarm, weil Wasserstoff nur in geringen Konzentrationen in entsprechende Kondensatströme übergeht. Er enthält jedoch Kohlenwasserstoffe mit zwei Kohlenstoffatomen sowie Methan. Wie zuvor erläutert, ist die Trennung eines Trenneinsatzstroms in einen C3-oder C3plus-Strom und einen C2- oder C2minus-Strom sehr aufwendig, wenn in dem Trenneinsatzstrom vergleichsweise große Mengen an Wasserstoff enthalten sind. Dieses Problem löst die vorliegende Erfindung durch die erläuterte Bildung entsprechender wasserstoffarmer Fraktionen (des ersten und zweiten Trennabstroms) aus einem Trenneinsatzstrom.

Ferner sieht die vorliegende Erfindung vor, dass aus Fluid des zweiten gasförmigen Reststroms ein zumindest Propylen und Propan enthaltender und an Wasserstoff abgereicherter, nicht notwendigerweise aber hiervon und von Methan freier, dritter Trennabstrom gebildet wird, und dass der Trenneinsatzstrom unter Verwendung von Fluid des dritten Trennabstroms gebildet wird. Bei der ersten und der zweiten Kondensation nicht in die Flüssigphase, also nicht in den ersten und den zweiten flüssigen Kondensatstrom übergehende, Anteile des in dem Trenneinsatzstrom enthaltenen Propylens und Propans werden also, ggf. zusammen mit Kohlenwasserstoffen mit zwei Kohlenstoffatomen, in Form des dritten Trennabstroms zurückgewonnen und in die Trennung zurückgeführt, also bei der Bildung des Trenneinsatzstroms verwendet. Zur Separation entsprechender Komponenten in dem zweiten gasförmigen Reststrom können die unten erläuterten Wege beschritten werden. Bei der Bildung des zumindest Propylen und Propan enthaltenden und an Wasserstoff abgereicherten dritten Trennabstroms wird zudem typischerweise ein zumindest Wasserstoff und Methan enthaltender, aber an Propylen und Propan armer vierter Trennabstrom gebildet. Kohlenwasserstoffe mit zwei Kohlenstoffatomen können, je nach spezifischer Ausgestaltung der Trennung, in den dritten oder vierten Trennabstrom oder beide übergehen.

Insgesamt lässt sich die vorliegende Erfindung zusammengefasst dadurch charakterisieren, dass aus einem entsprechenden Trenneinsatzstrom zunächst, d.h. in Form der Kondensationsschritte, soviel der jeweiligen Kohlenwasserstoffe mit drei Kohlenstoffatomen wie möglich aus einem entsprechend verdichteten Strom (z.B. bei 28 bar) auskondensiert werden. Aus den Kondensaten erfolgt eine Trennung in Kohlenwasserstoffe mit zwei Kohlenstoffatomen und Methan sowie den vergleichsweise geringen Mengen an Wasserstoff einerseits in Form des zweiten Trennabstroms und der Kohlenwasserstoffe mit drei (und mehr) Kohlenstoffatomen in Form des ersten Trennabstroms andererseits. Diese Trennung ist bei vergleichsweise hohen Kopftemperatruen (z.B. -35 °C) in einer Desti Ilationssäule nur dann mit hoher Trennschärfe möglich, wenn in der gebildeten Fraktion der Kohlenwasserstoffe mit zwei Kohlenstoffatomen, Methan und Wasserstoff, d.h. im zweiten Trennabstrom, das Verhältnis aus schwereren Komponenten (insbesondere der Kohlenwasserstoffe mit zwei Kohlenstoffatomen) und der leichteren Komponenten (insbesondere von Wasserstoff) tendenziell zugunsten der schwereren Komponenten ausfällt. Wie erläutert, kann dies durch die vorliegende Erfindung erreicht werden.

Nicht kondensiertes Fluid in Form des zweiten gasförmigen Reststroms kann gemäß einer besonders vorteilhaften Ausführungsform der Erfindung weiter auf ein viertes Temperaturniveau abgekühlt werden, beispielsweise auf -120 °C. Hierdurch werden praktisch sämtliche Kohlenwasserstoffe mit drei Kohlenstoffatomen in einen dritten flüssigen Kondensatstrom auskondensiert und eine wasserstoffreiche Gasphase verbleibt in Form eines dritten gasförmigen Reststroms. Die für diesen Abkühlvorgang benötigte Kälte kann durch die zuvor erläuterten Ströme selbst bzw. durch Entspannung auch nur bestimmter Teile hiervon bereitgestellt werden, wie auch nachfolgend noch im Detail erläutert.

Besonders vorteilhaft ist es in der erläuterten Ausführungsform, wenn zum Abkühlen des Fluids des ersten gasförmigen Reststroms ein zumindest mit Fluid des zweiten flüssigen Kondensatstroms gekühlter erster Gegenstromwärmetauscher und zum weiteren Abkühlen des Fluids des zweiten gasförmigen Reststroms ein zumindest mit Fluid des dritten flüssigen Kondensatstroms und des dritten gasförmigen Reststroms gekühlter zweiter Gegenstromwärmetauscher verwendet wird, wobei das Fluid des dritten flüssigen Kondensatstroms und ein Teil des Fluids des dritten gasförmigen Reststroms vor einem Einspeisen in den zweiten Gegenstromwärmetauscher entspannt wird (der übrige Teil des dritten gasförmigen Reststroms bildet den vierten Trennabstrom). Ein weiterer Teil des Fluids des dritten gasförmigen Reststroms kann auch ohne Entspannung durch den zweiten (und auch weitere) Gegenstromwärmetauscher geführt werden.

Ein wesentlicher Aspekt der vorliegenden Erfindung gemäß der erläuterten Ausführungsform ist dabei die Nutzung des zweiten gasförmigen Reststroms, der zum Teil entspannt und als Kältemittel zur Bereitstellung des dritten flüssigen Kondensatstroms und des dritten gasförmigen Reststroms auf dem vierten Temperaturniveau verwendet wird. Die Verwendung dieses zweiten gasförmigen Reststroms hat mehrere entscheidende Vorteile. Ein entsprechender Strom besitzt einen optimalen Effekt für einen Wärmetauscher, der zur Abkühlung auf das vierte Temperaturniveau verwendet wird. Durch die spezifische Führung der Fluide des dritten flüssigen Kondensat- und des dritten gasförmigen Reststroms wird eine besonders vorteilhafte Trennung erzielt.

Besonders günstig erweist es sich hierbei, wenn das Fluid des dritten flüssigen Kondensatstroms und der Teil des Fluids des dritten gasförmigen Reststroms, der entspannt wird, vor dem Einspeisen in den zweiten Gegenstromwärmetauscher mit Fluid des zweiten Trennabstroms zu einem Sammelstrom vereinigt wird und dieser nach einem Erwärmen in dem zweiten Gegenstromwärmetauscher in dem ersten Gegenstromwärmetauscher erwärmt wird.

Vorteilhafterweise wird also aus den in dem zweiten Gegenstromwärmetauscher verwendeten Kältemitteln, nämlich dem Fluid des dritten flüssigen Kondensatstroms und dem entspannten Teil des Fluids des dritten gasförmigen Reststroms ein gemischtes Kältemittel erzeugt, welches an jeder Stelle des Wärmetauschers eine zufriedenstellende Temperaturdifferenz gewährleisten kann.

Der Sammelstrom umfasst vorteilhafterweise auch einen Teil des zweiten Trennabstroms, der nach optionaler Abkühlung in dem zweiten Gegenstromwärmetauscher mit den Kältemitteln, d.h. dem Fluid des dritten flüssigen Kondensatstroms und dem entspannten Anteil des Fluids des dritten gasförmigen Reststroms, ebenfalls vermischt und danach zurückgeführt wird. Hierdurch werden die Temperaturdifferenzen im zweiten Gegenstromwärmetauscher nochmals positiv beeinflusst. Außerdem schafft dies einen deutlichen Vorteil bei der Destillation. Das Verhältnis von Kohlenwasserstoffatomen mit zwei Kohlenstoffatomen zu Wasserstoff in den ersten und zweiten flüssigen Kondensatströmen wird deutlich in Richtung von Kohlenwasserstoffen mit zwei Kohlenstoffatomen verschoben, was eine deutliche Temperaturerhöhung am Kopf einer verwendeten Destillationssäule und damit einen geringeren Kühlaufwand bzw. einen geringeren Aufwand an Kältemittel ermöglicht.

Zusammenfassend kann im Rahmen der vorliegenden Erfindung gemäß der erläuterten Ausführungsform der Verlust an Kohlenwasserstoffatomen mit drei Kohlenstoffatomen in eine C2- oder C2minus-Fraktion deutlich reduziert werden. Typische Werte sind beispielsweise 0,02% Verlust in den vierten Trennabstrom und 0,05% Verlust in den zweiten Trennabstrom. In Summe bleibt der Verlust unterhalb von 0,1%. Ein weiterer Vorteil der Erfindung gemäß der erläuterten Ausführungsform besteht darin, dass jeweils nur ein Wärmetauscher und ein Behälter verwendet wird, der bei Temperaturen von unter -40 °C arbeitet. Säm tliche Destillationen laufen oberhalb von -40 °C ab, was einen deutlichen Vortei I bei den Investitionskosten ergibt. Es können insbesondere kostengünstigere Materialien zum Einsatz kommen. Ferner brauchen keine aufwendigen und wartungsanfälligen Pumpen bei Temperaturen unterhalb von 0 °C zum Einsatz kommen. Der vierte T rennabstrom hat außerdem eine höhere Wasserstoffkonzentration (d.h. er weist beispielsweise einen Gehalt von 93 Mol-% anstelle von früher 88 Mol-% auf), da die meisten Nebenkomponenten in dem zweiten Trennabstrom landen. Dies führt zu Kostensenkungen bei der Wasserstoffgewinnung durch Druckwechseladsorption. Der zweite Trennabstrom wird ferner im Rahmen der vorliegenden Erfindung bei hohem Druck gewonnen. Dies kann insbesondere dann von Vorteil sein, wenn eine entsprechende Fraktion einer Olefingewinnung zugeführt oder der Strom in ein Fuelgassystem mit vergleichsweise hohem Druck eingeführt werden soll.

Neben dem soeben erläuterten Verfahren kann auch eine Ausführungsform besondere Vorteile bieten, bei der der zumindest Propylen und Propan enthaltende und an Methan und Wasserstoff arme dritte Trennabstrom sowie der erwähnte vierte Trennabstrom durch Druckwechseladsorption aus dem Fluid des zweiten gasförmigen Reststroms gebildet werden. Eine Druckwechseladsorption kann dazu dienen, auf die ansonsten noch erforderlichen tiefen Temperaturen zu verzichten.

In einem entsprechenden Verfahren werden vorteilhafterweise sämtliche Trennschritte auf einem einheitlichen überatmosphärischen Druckniveau durchgeführt, so dass keine aufwendige Entspannung und Rückverdichtung von Fluiden, insbesondere bei tiefen Temperaturen, erforderlich ist. Ein derartiges Druckniveau kann beispielsweise bei einem Absolutdruck von 10 bis 30 bar, insbesondere von 25 bis 30 bar, beispielsweise bei 28 bar, liegen. Es verringert sich im Verlauf der beschriebenen Trennsequenz geringfügig, beispielsweise um 1 bis 2 bar, wobei jedoch keine aktive Entspannung erfolgt. Der dritte Trennabstrom liegt allerdings letztlich typischerweise auf einem niedrigeren Druckniveau vor. Entweder wird dieser in der Druckwechseladsorption oder vor einer Verwendnung als Kältemittel auf einem Temperaturniveau von ca. -120 °C) entspannt. In beiden Fällen wird der dritte Trennabstrom beispielsweise auf 2 bar entspannt, die Entspannung kann jedoch auch auf ein höheres Druckniveau von 5 bis 10 bar, beispielsweise ca. 4,5 bar, erfolgen. Der dritte Trennabstrom kann bei der Verdichtung des Trenneinsatzstroms mitverdichtet und daher bei der Bildung des Trenneinsatzstroms verwendet werden.

Im Rahmen der vorliegenden Erfindung liegt das erste Temperaturniveau vorteilhafterweise bei 25 bis 40 °C, das zweite Temperaturniveau vorteilhafterweise bei 5 bis 10 °C und/oder das dritte Temperaturniveau vorteilhafterweise bei -30 bis -40 °C. Zur Abkühlung auf das zweite und das dritte Temperaturniveau kann beispielsweise Propan oder Propylen als Kältemittel zum Einsatz kommen, die Mittel zur Bereitstellung des vierten Temperaturniveaus, das beispielsweise bei -90 bis -130 °C liegen kann, wurden oben bereits erläutert.

Vorteilhafterweise wird also zum weiteren Abkühlen auf das dritte Temperaturniveau wenigstens ein Wärmetauscher verwendet, der mit Propan oder Propylen als Kältemittel betrieben wird. Es sind keine Kältemittel für Temperaturen unterhalb von - 40 °C erforderlich, die mittels eines gesonderten K ältemittelkreislaufes bereitgestellt werden müssten. Der erste Gegenstromwärmetauscher kann ferner mit Fluid des zweiten Trennabstroms gekühlt werden, in dem zweiten Gegenstromwärmetauscher kann, wie erwähnt, Fluid des zweiten Trennabstroms gekühlt werden. Vorteilhafterweise wird zur Bildung des ersten und des zweiten Trennabstroms aus dem Fluid des ersten und des zweiten flüssigen Kondensatstroms eine Destillationssäulenanordnung mit einer ersten und einer zweiten Destillationssäule verwendet.

Vorteilhafterweise wird dabei zumindest das Fluid des ersten flüssigen Kondensatstroms in die erste Destillationssäule eingespeist. Die erste Destillationssäule ist insbesondere als Deethanizer ausgebildet, so dass in dieser der erste Trennabstrom, der beträchtliche Mengen an Kohlenwasserstoffen mit drei Kohlenstoffatomen aufweist, abgetrennt werden kann. Der zweite flüssige Kondensatstrom kann hingegen in die zweite Destillationssäule eingespeist werden, da er bereits deutlich geringere Mengen an Kohlenwasserstoffen mit drei Kohlenstoffatomen aufweist. Andere Möglichkeiten zur Einspeisung können ebenfalls vorgesehen sein.

Vorteilhafterweise wird der erste Trennabstrom aus Fluid im Sumpf der ersten Destillationssäule und der zweite Trennabstrom aus Fluid am Kopf der zweiten Destillationssäule gebildet, wie nachfolgend erläutert. Der zweite Trennabstrom wird dabei vorteilhafterweise vom Kopf der zweiten Destillationssäule entnommen. Hierbei wird der zweite Trennabstrom in Form eines Fluids gebildet, das in einem Kopfkondensator der zweiten Destillationssäule bei -30 bis -35 °C gasförmig verbleibt. Wie erläutert, stellt die Verwendung derartiger, vergleichsweise hoher Temperaturen einen besonderen Vorteil der Erfindung dar. Auch der Kopfkondensator der zweiten Destillationssäule kann damit mit Propan oder Propylen als Kältemittel betrieben werden, es sind keine aufwändigen externen Kältemittel auf niedrigerem Temperaturniveau erforderlich. In einem entsprechenden Verfahren wird ferner Fluid vom Kopf der ersten Destillationssäule entnommen, abgekühlt und anschließend in die zweite Destillationssäule überführt.

Eine vorteilhafte Ausgestaltung des erfindungsgemäßen Verfahrens sieht vor, dass die erste Destillationssäule und die zweite Destillationssäule der Destillationssäulenanordnung als Teil einer Doppelsäule ausgebildet sind, in der die zweite Destillationssäule oberhalb der ersten Destillationssäule angeordnet ist und die beiden Destillationssäulen in der Doppelsäule baulich miteinander verbunden sind, beispielsweise eine gemeinsame Außenhülle aufweisen. Dies kann beispielsweise die Erstellung einer entsprechenden Anlage vereinfachen.

Eine weitere vorteilhafte Ausgestaltung des Verfahrens sieht vor, dass das Fluid des ersten und/oder zweiten flüssigen Kondensatstroms vor dem Einspeisen in die Destillationssäulenanordnung durch Strippen weiter an Wasserstoff abgereichert wird. Eine erste Abreicherung ist bereits durch die Bildung des ersten bzw. zweiten flüssigen Kondensatstroms selbst erfolgt, die dessen Wasserstoffgehalt gegenüber dem Trenneinsatzstrom verringert. Das Strippen verringert den Trennaufwand nochmals.

In diesem Zusammenhang kann eine Variante des Verfahrens besonders vorteilhaft sein, bei der das Fluid des ersten flüssigen Kondensatstroms vor dem Strippen mit Fluid aus dem Sumpf der zweiten Destillationssäule vereinigt wird, da hierdurch eine weitere Wasserstoffabreicherung aus dem Destillationssystem erfolgen kann.

Die vorliegende Erfindung bezieht sich auch auf eine Anlage zur Gewinnung von Propylen, zu deren Merkmalen und Vorteilen auf den entsprechenden Patentanspruch bzw. die obigen Erläuterungen verwiesen wird. Eine entsprechende Anlage profitiert von den zuvor erläuterten Vorteilen. Insbesondere ist eine entsprechende Anlage zur Durchführung eines Verfahrens eingerichtet, wie es zuvor erläutert wurde.

Die Erfindung wird nachfolgend unter Bezugnahme auf die beigefügten Zeichnungen näher erläutert, die bevorzugte Ausführungsformen der Erfindung unter Bezugnahme auf den Stand der Technik veranschaulichen.

### Kurze Beschreibung der Zeichnungen

Figur 1 veranschaulicht ein Verfahren zur Gewinnung von Propylen in Form eines schematischen Ablaufplans.
Figur 2 veranschaulicht Details eines Verfahrens zur Gewinnung von Propylen in Form eines schematischen Ablaufplans.
Figur 3 veranschaulicht Details eines Verfahrens zur Gewinnung von Propylen gemäß einer Ausführungsform der Erfindung in Form eines schematischen Ablaufplans.
Figur 4 veranschaulicht Details eines Verfahrens zur Gewinnung von Propylen gemäß einer Ausführungsform der Erfindung in Form eines schematischen Ablaufplans.
Figur 5 veranschaulicht Details eines Verfahrens zur Gewinnung von Propylen gemäß einer Ausführungsform der Erfindung in Form eines schematischen Ablaufplans.
Figur 6 veranschaulicht Details eines Verfahrens zur Gewinnung von Propylen gemäß einer Ausführungsform der Erfindung in Form eines schematischen Ablaufplans.
Figur 7 veranschaulicht eine zum Einsatz gemäß einer Ausführungsform der Erfindung vorteilhafte Doppelsäule in schematischer Darstellung.
Figur 8 veranschaulicht eine zum Einsatz gemäß einer Ausführungsform der Erfindung vorteilhafte Doppelsäule in schematischer Darstellung.

In den Figuren sind einander entsprechende Elemente, Anlagenteile und Ströme mit einander entsprechenden Bezugszeichen veranschaulicht und werden der Übersichtlichkeit halber nicht wiederholt erläutert.

### Ausführliche Beschreibung der Zeichnungen

In Figur 1 ist eine Anlage zur Gewinnung von Propylen in Form eines schematischen Ablaufplans veranschaulicht und insgesamt mit 100 bezeichnet.

In dem Verfahren 100 wird ein Einsatzstrom A einem Dehydrierungsschritt 101 unterworfen und in dem Dehydrierungsschritt zusammen mit einem Dampfstrom B zu einem Produktstrom C, der an dieser Stelle auch als Rohgasstrom bezeichnet werden kann, umgesetzt. Bei dieser Umsetzung wird ein Teil des in dem Einsatzstrom enthaltenen Propans (der Einsatzstrom A ist reich an Propan oder besteht ausschließlich aus Propan) zu Propylen umgesetzt. Dem Dehydrierungsschritt 101 ist ein Katalysatorregenerationsschritt 102 zugeordnet, der der Übersichtlichkeit halber nicht erläutert wird. Zur Bereitstellung des Dampfstroms B ist ein Dampfbereitstellungsschritt 103 bzw. ein entsprechendes Dampferzeugungssystem vorgesehen. Dieses kann beispielsweise auch mit einem Quenchwasserstrom D gespeist werden, wie nachfolgend noch erläutert.

Der Produktstrom C (Rohgasstrom) wird einem Kühlschritt 104 unterworfen. Ein entsprechend abgekühlter Strom, nun mit E bezeichnet, wird anschließend einem Quenchschritt 105 zugeführt, in dem der Strom E in direkten Kontakt mit einem Kühlwasserstrom gebracht wird. Der Kühlwasserstrom ist der Übersichtlichkeit halber nicht veranschaulicht. In dem Quenchschritt 105 wird zuvor in Form des Dampfstroms B zugeführter Prozessdampf aus dem Strom E auskondensiert. Ein hierbei erhaltener Quenchwasserstrom, wie bereits erwähnt mit D bezeichnet, kann dem Dampferzeugungsschritt 103 unterworfen werden. Ein auf diese Weise zum Teil von Wasser befreiter Strom, nun mit F bezeichnet, wird anschließend einem Verdichtungsschritt 106 unterworfen, der mit einem Sauergasentfernungsschritt 107 gekoppelt sein kann. Details werden auch hier nicht erläutert.

Nach der Verdichtung liegt ein hier als Trenneinsatzstrom bezeichneter Strom G vor und wird mehreren nachfolgenden Schritten zugeführt. Zunächst wird der Trenneinsatzstrom G dabei einem Vorkühl- und Trocknungsschritt 108 unterworfen, in welchem ein überwiegend Kohlenwasserstoffe mit zwei Kohlenstoffatomen, Methan und Wasserstoff enthaltender Strom H anfällt. Der Strom H enthält jedoch zudem noch Kohlenwasserstoffe mit drei und gegebenenfalls mehr Kohlenstoffatomen, welche in einem Niedertemperaturtrennschritt 109 in Form des Stroms K abgetrennt (rückgewonnen) werden können. Es verbleiben ein oder mehrere Restströme, wie hier mit O und P angegeben. Hierbei kann es sich beispielsweise um im Wesentlichen reinen Wasserstoff, jedoch auch um Gemische aus Wasserstoff und Methan und gegebenenfalls leichteren Kohlenwasserstoffen mit zwei Kohlenstoffatomen handeln.

Ein Strom I, der ebenfalls in dem Vorkühl- und Trocknungsschritt 108 anfällt und überwiegend Kohlenwasserstoffe mit zwei und mehr Kohlenstoffatomen enthält, wird zusammen mit dem erwähnten Strom K einem Deethanizerschritt 110 zugeführt. Der Deethanizerschritt dient im Wesentlichen zur Gewinnung eines an Kohlenwasserstoff mit zwei Kohlenstoffatomen reichen und an Kohlenwasserstoffen mit drei und mehr Kohlenstoffatomen armen Stroms T, der hier erneut dem Vorkühl- und Trocknungsschritt 108 unterworfen wird. Ein verbleibender Strom S enthält überwiegend Kohlenwasserstoffe mit drei und mehr Kohlenstoffatomen und ist arm an Kohlenwasserstoffen mit zwei Kohlenstoffatomen, Methan und Wasserstoff.

Der Strom S wird einem Produkttrennschritt 111 zugeführt, in welchem im Wesentlichen Propylen in Form des Stroms U aus dem Strom S abgetrennt wird. Der Produkttrennschritt 111 kann hierzu mit einem Propylenkältemittelkreislauf 112 gekoppelt sein. Ein verbleibender Strom V enthält überwiegend Kohlenwasserstoffe mit drei und mehr Kohlenstoffatomen, nachdem das Propylen in Form des Stroms U abgetrennt wurde. Der Strom V wird einem weiteren Trennschritt 113 zugeführt, dem typischerweise auch ein propanreicher Strom X als sogenannter Frischfeed zugespeist wird. Kohlenwasserstoffe mit vier und mehr Kohlenstoffatomen werden in Form des Stroms W aus dem Strom V und X abgetrennt. Ein verbleibender Strom Y, der noch einen gewissen Anteil an Kohlenwasserstoffen mit drei Kohlenstoffatomen in ungesättigter Form enthalten kann, wird einer Hydrierungseinheit 114 zugeführt. Stromab der Hydrierungseinheit 114 liegt der bereits erwähnte Strom A vor, bei dem es sich im Wesentlichen um reines Propan handelt.

In Figur 2 sind Details eines Verfahrens zur Gewinnung von Propylen veranschaulicht, wobei in der Darstellung der Figur 2 insbesondere die Komponenten 108, 109 und 110 des in Figur 1 veranschaulichten Verfahrens 100, also der Vorkühl- und Trocknungsschritt 108, der Tieftemperaturtrennschritt 109 und der Deethanizerschritt 110 veranschaulicht sind. Die Trennung der einzelnen Schritte ist nicht durch die Darstellung in der Zeichnung festgelegt.

Der Trenneinsatzstrom G, der stromauf, wie erläutert, auf eine zur Trennung geeignete Temperatur abgekühlt und zuvor entsprechend verdichtet worden sein kann, wird im dargestellten Beispiel aus dem erwähnten Strom F gebildet und beispielsweise auf einem Druck von 28 bar und einer Temperatur von 37 °C, die mittels eines Verdichters 1 bzw. eines Wärmetauschers 2 erhalten wurden, einem weiteren Wärmetauscher 3 zugeführt. Der Strom G wird in dem weiteren Wärmetauscher 3 von der zuvor erläuterten Temperatur, die hier als erstes Temperaturniveau bezeichnet wird, auf eine Temperatur von beispielsweise +10 °C, die hier als zweites Temperaturniveau bezeichnet wird, abgekühlt. Zur Abkühlung in dem Wärmetauscher 3 wird beispielsweise Propylen als Kältemittel verwendet.

Der entsprechend abgekühlte Strom wird anschließend in einen Abscheidebehälter 4 überführt, in welchem ein "erster" flüssiger Kondensatstrom in Form des Stroms I sowie ein "erster" gasförmiger Reststrom in Form des Stroms H erhalten werden. Die Ströme I und H sind bereits in der Figur 1 veranschaulicht. Aus dem Strom I wird in einem Wasserabscheider 5 Wasser abgeschieden (der Wasserstrom ist mit U bezeichnet) und der Strom I wird anschließend mittels einer Pumpe 6 einem adsorptiven Trockner 7 zugeführt. Entsprechend wird auch der Strom H einem adsorptiven Trockner 8 zugeführt. Der Strom H wird anschließend in eine Doppelsäule 9 überführt, in welcher der bereits zuvor erläuterte Strom K erhalten und mittels einer Pumpe 10 in eine Deethanizersäule 11 überführt wird.

Der Deethanizersäule 11 wird ferner der Strom I zugeführt, der nach der Trocknung in dem adsorptiven Trockner 7 in einem Wärmetauscher 12 erwärmt wurde. In der Deethanizersäule 11, die einen Sumpfverdampfer 13 und einen Kopfkondensator 14 aufweist, wird bodenseitig der bereits mehrfach erläuterte Strom S ("erster Trennabstrom") gewonnen, also ein Strom, der überwiegend Kohlenwasserstoffe mit drei und mehr Kohlenstoffatomen aufweist. Kopfseitig wird ein gasförmiger Strom abgezogen. Ein nicht in dem Kopfkondensator 14 kondensierender Anteil hiervon wird in Form des ebenfalls erläuterten Stroms T nach Abkühlung in Wärmetauscher 12 mit dem Strom H vereinigt. Die Deethanizersäule 11 wird im Sumpf bei beispielsweise 70 °C und einem Druck von beispielsweise 28 bar und in dem Kopfkondensator 14 bei einer Temperatur von beispielsweise 37 °C betrieben.

Vom Kopf der beiden Teile der Doppelsäule 9 werden jeweils Ströme L und M abgezogen und in Wärmetauschern 15 bzw. 16 teilverflüssigt. Der Strom L wird in einen unteren Bereich des oberen Teils der Destillationssäule 9 eingespeist, der Strom M am Kopf des oberen Teils oberhalb einer Flüssigkeitsabdichtung der Destillationssäule 9 aufgegeben. Aus dem Sumpf des oberen Teils der Destillationssäule 9 wird ein Strom N abgezogen und in den unteren Teil der Destillationssäule 9 überführt. Außerdem wird ein Recyclestrom P aus dem Sumpf des oberen Teils der Destillationssäule 9 abgezogen und ggf. in Wärmetauscher 15 abgekühlt. Nach Entspannung wird dieser Recyclestrom P wieder in Wärmetauscher 15 angewärmt. Oberhalb der Flüssigkeitsabdichtung am Kopf des oberen Teils der Destillationssäule 9 wird ferner ein gasförmiger Strom abgezogen, durch den Wärmetauscher 15 geführt und als Tailgasstrom O ausgeführt. Ein Teil des Tailgasstroms O kann ggf. vor Anwärmung in den Recyclestrom P gemischt werden. Der Tailgasstrom O kann beispielsweise überwiegend Wasserstoff enthalten, der Recyclestrom P beispielsweise Kohlenwasserstoffe mit drei Kohlenstoffatomen, zwei Kohlenstoffatomen, Methan und Wasserstoff. Der Recyclestrom P kann damit mit einer vergleichbaren Zusammensetzung bereitgestellt werden wie der "dritte Trennabstrom" gemäß der vorliegenden Erfindung, der Tailgasstrom O mit einer Zusammensetzung, die vergleichbar mit dem "vierten Trennabstrom" der vorliegenden Erfindung sein kann.

Im dargestellten Beispiel wird der Wärmetauscher 16 mittels zweier Kältekreisläufe Q und R betrieben, wobei sich bei dem Kältekreislauf Q um einen Kältekreislauf mit Propylen auf einer Temperatur von -20 °C und bei de m Kältekreislauf R um einen Kältekreislauf mit Propylen auf -40 °C handeln kann. Zusätzlich werden durch den Wärmetauscher 16 der Recyclestrom O und der Tailgasstrom P geführt. Der Recyclestrom O und der Tailgasstrom P werden in Wärmetauscher 12 weiter erwärmt.

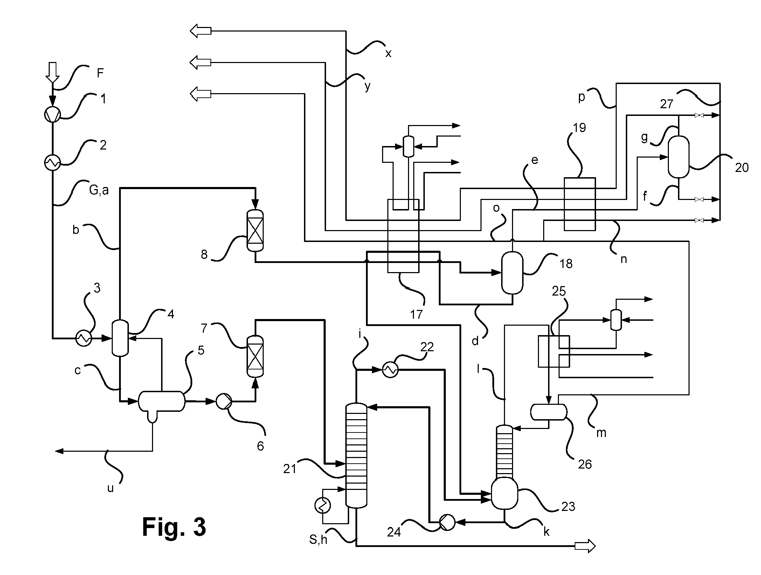

In Figur 3 sind Details eines Verfahrens zur Gewinnung von Propylen gemäß einer Ausführungsform der Erfindung schematisch veranschaulicht. Zur besseren Unterscheidbarkeit sind hier die jeweiligen Ströme mit Kleinbuchstaben bezeichnet.

Ein Trenneinsatzstrom G, der hier auch mit a bezeichnet wird, wird ebenfalls durch einen Verdichter 1 und einen Wärmetauscher 2 geführt. Wie zuvor, liegt dieser anschließend auf dem ersten Temperaturniveau vor. Auch hier wird dieser Strom durch einen weiteren Wärmetauscher 3 geführt und anschließend in einen ("ersten") Abscheidebehälter 4 auf dem zweiten Temperaturniveau eingespeist. Auch die Abscheidung von Wasser (Wasserstrom u) aus dem flüssigen Kondensatstrom, der hier mit c bezeichnet ist ("erster" Kondensatstrom, vergleiche Strom I gemäß Figur 2) in dem Wasserabscheider 5 erfolgt in vergleichbarer Weise, ebenso wie die zunächst weitere Behandlung des Stroms c in der Pumpe 6 und dem adsorptiven Trockner 7. Entsprechendes gilt auch für den hier mit b bezeichneten Restgasstrom ("erster" Restgasstrom, vergleiche Strom H gemäß Figur 2).

Der Strom b ("erster" gasförmiger Reststrom) wird hier jedoch anschließend durch einen ("ersten") Gegenstromwärmetauscher 17 geführt und, nach Abkühlung auf ein drittes Temperaturniveau, das beispielsweise bei -37 °C liegt, in einen weiteren ("zweiten") Abscheidebehälter 18 überführt. Der Druck an dieser Stelle beträgt beispielsweise 27 bar. In dem Abscheidebehälter 18 wird ein "zweiter" flüssiger Kondensatstrom d gewonnen, der in dem Gegenstromwärmetauscher 17 als Kältemittel verwendet wird. Der Gegenstromwärmetauscher 17 kann, ähnlich wie ein Wärmetauscher 25 bzw. der Wärmetauscher 16 gemäß Figur 2, mit zwei Propylen-bzw. C3-Kältemittelkreisläufen betrieben werden. Am Kopf des Abscheidebehälters 18 fällt ein "zweiter" gasförmiger Reststrom e an, der, ausgehend von dem dritten Temperaturniveau, in einem weiteren ("zweiten") Gegenstromwärmetauscher 19 auf ein viertes Temperaturniveau abgekühlt wird. Das vierte Temperaturniveau liegt beispielsweise bei -120 °C, der Druck an dieser Ste Ile beträgt beispielsweise 26,9 bar. Nach Abkühlen auf das vierte Temperaturniveau wird der Strom e in einen weiteren ("dritten") Abscheidebehälter 20 überführt, in welchem ein "dritter" flüssiger Kondensatstrom f und ein "dritter" gasförmiger Reststrom g erhalten werden.

Zunächst wird nun die weitere Trennung des Stroms c gemäß Figur 3 erläutert. Der Strom c, also der erste flüssige Kondensatstrom, wird dabei nach Abtrennung des Wassers in dem Wasserabscheider 5 und weiterer Trocknung in dem adsorptiven Trockner 7 in eine Deethanizersäule 21 überführt, in der Fluid des Stroms c aufgetrennt wird. Die Deethanizersäule 21, die hier auch als "erste Destillationssäule" bezeichnet wird, wird mit einem nicht näher bezeichneten Sumpfverdampfer und beispielsweise auf einer Temperatur von 70 °C und einem Druck von 28 bar betrieben.

Im Sumpf der Deethanizersäule 21 fällt ein Sumpfprodukt an, das in Form eines Propylen und Propan enthaltenden, an Kohlenwasserstoffen mit zwei Kohlenstoffatomen, Methan und Wasserstoff armen, jedoch gegebenenfalls noch Kohlenwasserstoffe mit vier und mehr Kohlenstoffatomen enthaltenden Stroms h aus der Deethanizersäule 21 abgezogen wird. Der Strom h entspricht damit im Wesentlichen dem Strom S gemäß den Figuren 1 und 2 und wird hier wie oben auch als "erster Trennabstrom" bezeichnet.

Vom Kopf der Deethanizersäule 21 wird ein Strom i abgezogen, durch einen Wärmetauscher 22 geführt und hier auf eine Temperatur von beispielsweise 37 °C abgekühlt. Der Strom i wird anschließend in eine zweite Destillationssäule 23 überführt, in welche auch der zweite flüssige Kondensatstrom d aus dem Abscheidebehälter 18 eingespeist wird. Aus dem Sumpf der zweiten Destillationssäule 23 wird ein Strom k abgezogen und mittels einer Pumpe 24 in die Deethanizersäule 21 zurückgeführt. Vom Kopf der zweiten Destillationssäule 23 wird ein gasförmiger Strom I abgezogen und durch den Wärmetauscher 25 geführt, der mit Propylen- bzw. C3-Kältemittel wie zuvor unter Bezugnahme auf den Wärmetauscher 16 der Figur 2 beschrieben gekühlt wird. In einem Abscheidebehälter 26 der zweiten Destillationssäule 23 wird ein flüssiger Strom gewonnen und als Rücklauf auf die zweite Destillationssäule 23 zurückgeführt. Ein in dem Kopfkondensator bei beispielsweise -31 °C und 26 bar gasförmig verbleibendes Fluid wird in Form des Stroms m bereitgestellt. Der Strom m wird hier auch als "zweiter Trennabstrom" bezeichnet.

Ein Teil des Stroms m, der aufgrund seiner Bildung aus dem ersten flüssigen Kondensatstrom c und dem zweiten flüssigen Kondensatstrom d vergleichsweise geringe Mengen an Wasserstoff enthält, jedoch reich an Kohlenwasserstoffen mit zwei Kohlenstoffatomen ist, wird in Form des Stroms n abgezweigt, der Rest in Form des Stroms o durch den Wärmetauscher 17 geführt und als entsprechender Hochdruckstrom bereitgestellt.

Der dritte flüssige Kondensatstrom f aus dem Sumpf des Abscheidebehälters 20, ein Teil des dritten gasförmigen Reststroms g vom Kopf dieses Abscheidebehälters 20 und der Strom n werden in eine Sammelleitung 27 entspannt, wodurch ein gemischtes Kältemittel in der Sammelleitung 27 erhalten wird. Dieses gemischte Kältemittel wird in Form des Stroms p durch den zweiten Gegenstromwärmetauscher 19 und den ersten Gegenstromwärmetauscher 17 zurückgeführt. Es wird als Strom x bereitgestellt ("dritter Trennabstrom") und kann vollständig oder teilweise der dargestellten Trennung erneut zugeführt werden, beispielsweise indem es stromauf des Verdichters 2 mit dem Strom F vereinigt und damit bei der Bildung des Stroms G bzw. a verwendet wird.

Entsprechend wird der nicht entspannte Rest des gasförmigen Reststroms g vom Kopf des Abscheidebehälters 20 durch den zweiten Gegenstromwärmetauscher 19 und den ersten Gegenstromwärmetauscher 17 zurückgeführt. Dieser Strom ist mit y bezeichnet ("vierter Trennabstrom") und kann beispielsweise als rohes Wasserstoffprodukt bereitgestellt werden.

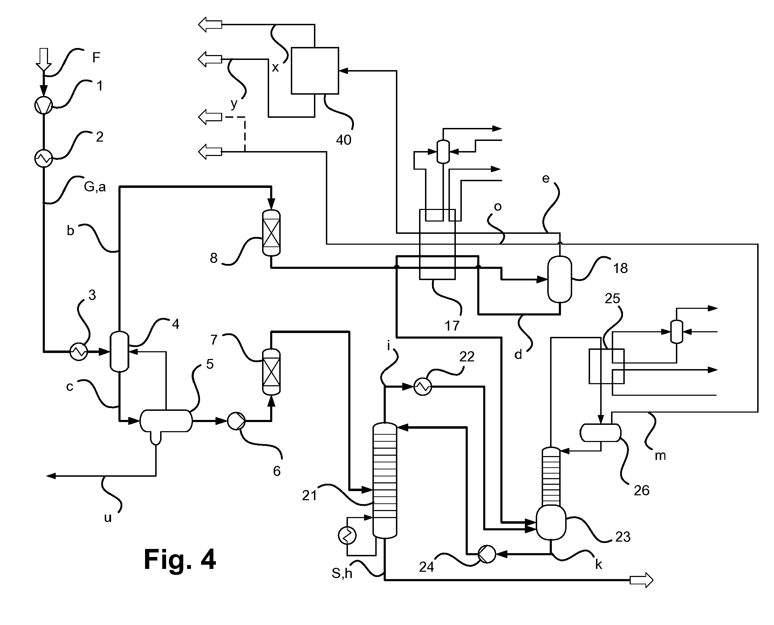

Figur 4 veranschaulicht Details eines Verfahrens zur Gewinnung von Propylen gemäß einer weiteren Ausführungsform der Erfindung in Form eines schematischen Ablaufplans. Die Bereitstellung der Ströme c und d und deren Trennung erfolgt hier grundsätzlich vergleichbar zu dem in Figur 3 veranschaulichten Verfahren und wird daher nicht erneut erläutert.

Der zweite gasförmige Reststrom, also Strom e, wird gemäß dem in Figur 4 veranschaulichten Verfahren jedoch nicht weiter abgekühlt, sondern in dem ("ersten") Gegenstromwärmetauscher 17 erwärmt und anschließend einem Druckwechseladsorptionsschritt 40 unterworfen. In dem Druckwechseladsorptionsschritt 40 können der Strom x und der Strom y ("dritter Trennabstrom" und "vierter Trennabstrom") im Wesentlichen in der gleichen oder einer vergleichbaren Zusammensetzung gewonnen werden, wie unter Bezugnahme auf die Figur 3 erläutert. Die genannten Ströme können wie dort erläutert behandelt werden.

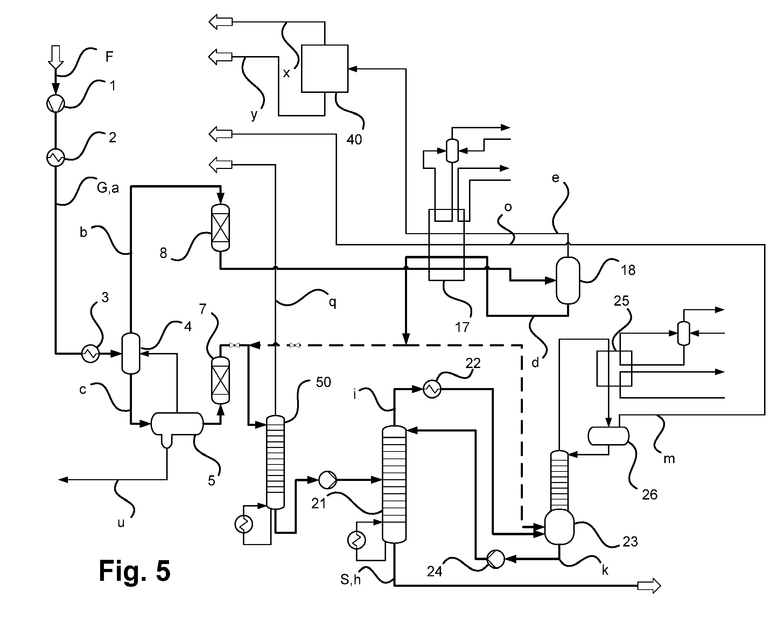

Figur 5 veranschaulicht Details eines Verfahrens zur Gewinnung von Propylen gemäß einer weiteren Ausführungsform der Erfindung in Form eines schematischen Ablaufplans. Das in Figur 5 veranschaulichte Verfahren stellt eine Variante des in Figur 4 veranschaulichten Verfahrens dar. Gemeinsamkeiten werden nicht erläutert.

Das in Figur 5 veranschaulichte Verfahren umfasst zusätzlich die Verwendung einer Strippersäule 50, in der in dem Strom g enthaltener Wasserstoff zumindest teilweise ausgestrippt und in Form des Stroms q ausgeführt werden kann. Der Strom d ("zweiter flüssiger Kondensatstrom") kann wahlweise zusammen mit dem Strom c in die Strippersäule 50 und/oder in die zweite Destillationssäule 23 eingespeist werden.

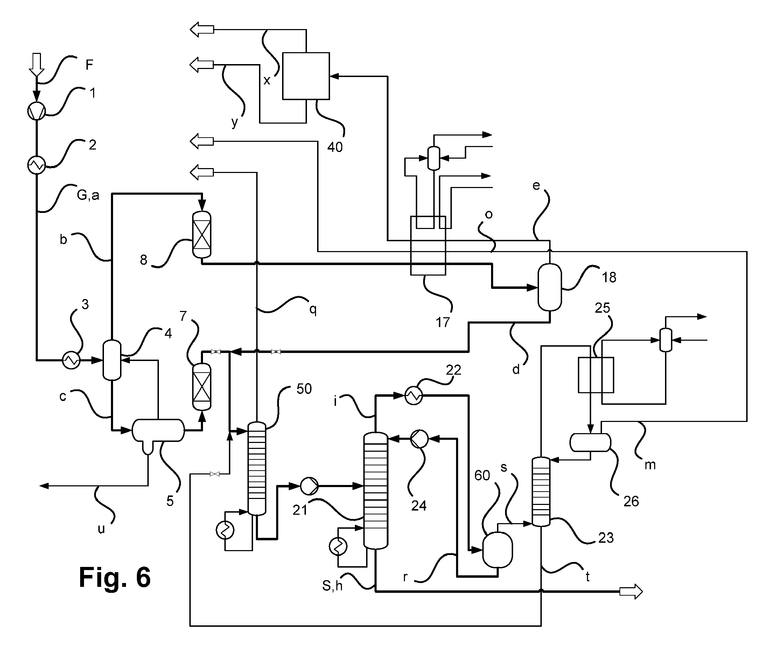

Figur 6 veranschaulicht Details eines Verfahrens zur Gewinnung von Propylen gemäß einer weiteren Ausführungsform der Erfindung in Form eines schematischen Ablaufplans. Das in Figur 6 veranschaulichte Verfahren stellt eine Variante des in Figur 5 veranschaulichten Verfahrens dar. Gemeinsamkeiten werden nicht erläutert.

Das in Figur 6 veranschaulichte Verfahren umfasst zusätzlich die Verwendung eines Abscheidebehälters 60 zwischen der ersten Destillationssäule 21 und der zweiten Destillationssäule 23, in den der Strom i eingespeist wird. Dem Abscheidebehälter 60 entnommene Flüssigkeit wird in Form des Stroms r in die erste Destillationssäule 21 zurückgeführt, dem Abscheidebehälter 60 entnommenes Gas wird in Form des Stroms s in die zweite Destillationssäule 23 eingespeist. Aus dem Sumpf der zweiten Destillationssäule 23 wird ein Strom t abgezogen und stromauf der Strippersäule 50 mit dem Strom c vereinigt. Der Strom d ("zweiter flüssiger Kondensatstrom") wird hier zusammen mit dem Strom c und dem Strom t in die Strippersäule 50 eingespeist.

Es versteht sich, dass sämtliche unter Bezugnahme auf bestimmte Ausführungsformen erläuterten Ausgestaltungen auch bei den jeweils anderen Ausführungsformen zum Einsatz kommen können, selbst wenn dies nicht explizit gezeigt ist.

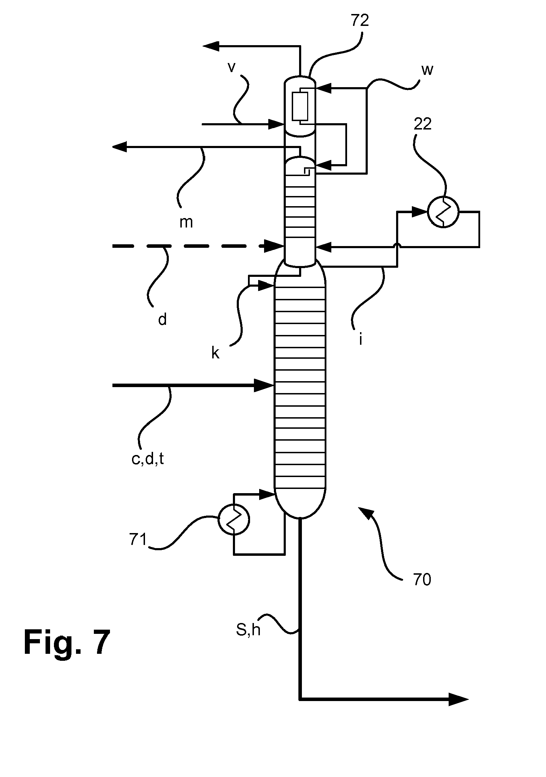

Figur 7 veranschaulicht eine zum Einsatz gemäß einer Ausführungsform der Erfindung vorteilhafte Doppelsäule in schematischer Darstellung.

Die erste Destillationssäule 21 und die zweite Destillationssäule 23 der in den Figuren 3 bis 6 veranschaulichten Ausführungsformen stellen eine Destillationssäulenanordnung dar, die auch als Doppelsäule ausgebildet sein kann, deren unterer Teil der ersten Destillationssäule 21 und deren oberer Teil der zweiten Destillationssäule 23 entsprechen kann.

Eine in Figur 7 gezeigte Doppelsäule ist mit 70 bezeichnet. Ein Sumpfverdampfer 71 der Doppelsäule 70 kann, wie auch die Sumpfverdampfer der ersten Destillationssäule 21 der zuvor erläuterten Ausführungsformen, mit Niederdruckdampf und/oder mit einem Quenchwasserstrom D (vgl. Figur 1) beheizt werden. In den unteren Teil der Doppelsäule 70 können die Ströme c (siehe Figuren 3 bis 6), d (siehe Figur 5, ggf. nur teilweise) und t (siehe Figur 6) eingespeist werden, in den oberen Teil der Strom d (siehe Figur 5, ggf. nur teilweise). Dem Sumpf des unteren Teils der Doppelsäule 70 wird der erste Produktstrom S bzw. h entnommen. Aus einem oberen Bereich des unteren Teils der Doppelsäule 70 kann ein gasförmiger Strom (auch hier mit i bezeichnet) entnommen, in einem Wärmetauscher (auch hier mit 22 bezeichnet) gekühlt und in den oberen Teil der Doppelsäule 70 überführt werden. Aus einem unteren Bereich des oberen Teils der Doppelsäule 70 kann ein Strom (auch hier mit k bezeichnet) in den oberen Bereich des unteren Teils zurückgeführt werden.

Aus einem oberen Bereich des oberen Teils der Doppelsäule 70 (oberhalb einer Flüssigkeitsabdichtung) wird ein dem Strom m der zuvor erläuterten Ausführungsformen entsprechender Strom, hier ebenfalls mit m bezeichnet, entnommen. Einem Verdampfungsraum eines Kopfkondensators 72 der Doppelsäule 70 wird ein geeignetes C3-Kältemittel zugeführt und diesem in verdampfter Form entnommen. In einem Verflüssigungsraum des Kopfkondensators 72 wird ein aus einem oberen Bereich des oberen Teils der Destillationssäule 70 (untererhalb der Flüssigkeitsabdichtung) abgezogener Strom w verflüssigt, welcher anschließend als Rücklauf auf den oberen Teil der Destillationssäule 70 (oberhalb der Flüssigkeitsabdichtung) aufgegeben wird.

Die Doppelsäule 70 entspricht in ihrer Funktionsweise im Wesentlichen den in den Figuren 1 bis 5 veranschaulichten Destillationssäulenanordnungen.

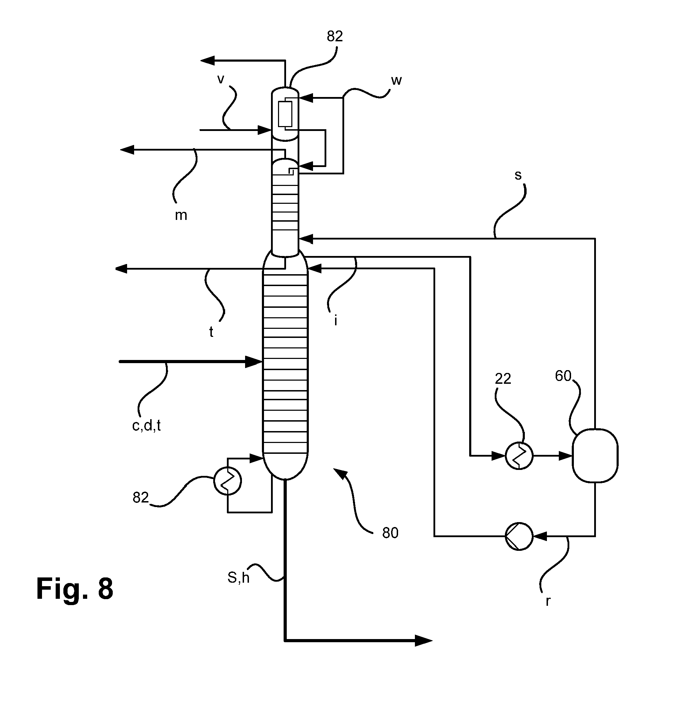

Figur 8 veranschaulicht eine weitere zum Einsatz gemäß einer Ausführungsform der Erfindung vorteilhafte Doppelsäule in schematischer Darstellung, die insgesamt mit 80 bezeichnet ist und in ihrer Funktionsweise im Wesentlichen der in der Figur 6 veranschaulichten Destillationssäulenanordnung entspricht. Ein Sumpfverdampfer 81 der Doppelsäule 80 ist mit 81, ihr Kopfkondensator mit 82 bezeichnet.

Zu weiteren Merkmalen der Doppelsäule 80, insbesondere der hier identisch bezeichneten Ströme, sei ausdrücklich auf die Erläuterungen zu Figur 6 verwiesen.

## Patentansprüche

1. Verfahren (100) zur Gewinnung von Propylen, bei dem ein Trenneinsatzstrom (G) bereitgestellt wird, der in einem Dehydrierungsschritt (101) aus Propan gebildetes Propylen sowie Propan, Kohlenwasserstoffe mit zwei Kohlenstoffatomen, Methan und Wasserstoff enthält, wobei aus Fluid des Trenneinsatzstroms (G) auf einem überatmosphärischen Druckniveau ein Propan und Propylen enthaltender und an Kohlenwasserstoffen mit zwei Kohlenstoffatomen, Methan und Wasserstoff armer erster Trennabstrom (S) gebildet wird, **dadurch gekennzeichnet, dass**
a) aus Fluid des Trenneinsatzstroms (G) nach Abkühlen von einem ersten auf ein zweites Temperaturniveau ein erster flüssiger Kondensatstrom (c) und ein erster gasförmiger Reststrom (b) gebildet werden,
b) aus Fluid des ersten gasförmigen Reststroms (b) nach weiterem Abkühlen auf ein drittes Temperaturniveau ein zweiter flüssiger Kondensatstrom (d) und ein zweiter gasförmiger Reststrom (e) gebildet werden,
c) aus Fluid des ersten (c) und des zweiten flüssigen Kondensatstroms (d) der erste (S) und ein Methan und Kohlenwasserstoffe mit zwei Kohlenstoffatomen enthaltender und an Wasserstoff und Kohlenwasserstoffen mit mehr als drei Kohlenstoffatomen armer zweiter Trennabstrom (m) gebildet werden,
d) aus Fluid des zweiten gasförmigen Reststroms (e) ein zumindest Propylen und Propan enthaltender und an Wasserstoff abgereicherter dritter Trennabstrom (x) gebildet wird, und
e) der Trenneinsatzstrom (G) unter Verwendung von Fluid des dritten Trennabstroms (x) gebildet wird.

2. Verfahren nach Anspruch 1, bei dem aus Fluid des zweiten gasförmigen Reststroms (e) nach weiterem Abkühlen auf ein viertes Temperaturniveau von -90 bis -130 °C ein dritter flüssiger Kondensatstrom (f) und ein dritter gasförmiger Reststrom (g) gebildet werden.

3. Verfahren nach Anspruch 2, bei dem zum Abkühlen des Fluids des ersten gasförmigen Reststroms (b) ein zumindest mit Fluid des zweiten flüssigen Kondensatstroms (d) gekühlter erster Gegenstromwärmetauscher (17) und zum weiteren Abkühlen des Fluids des zweiten gasförmigen Reststroms (e) ein zumindest mit Fluid des dritten flüssigen Kondensatstroms (f) und des dritten gasförmigen Reststroms (g) gekühlter zweiter Gegenstromwärmetauscher (19) verwendet wird, wobei das Fluid des dritten flüssigen Kondensatstroms (f) und ein Teil des Fluids des dritten gasförmigen Reststroms (g) vor einem Einspeisen in den zweiten Gegenstromwärmetauscher (19) entspannt wird.

4. Verfahren nach Anspruch 3, bei dem das Fluid des dritten flüssigen Kondensatstroms (f) und der Teil des Fluids des dritten gasförmigen Reststroms (g) vor dem Einspeisen in den zweiten Gegenstromwärmetauscher (19) mit Fluid des zweiten Trennabstroms (m) zu einem Sammelstrom (p) vereinigt wird, der nach einem Erwärmen in dem zweiten Gegenstromwärmetauscher (19) in dem ersten Gegenstromwärmetauscher (17) erwärmt wird.

5. Verfahren nach Anspruch 1, bei dem der zumindest Propylen und Propan enthaltende und an Wasserstoff abgereicherte dritte Trennabstrom (x) sowie ein zumindest Wasserstoff und Methan enthaltender und an Propylen und Propan armer vierter Trennabstrom (y) durch Druckwechseladsorption aus dem Fluid des zweiten gasförmigen Reststroms (e) gebildet werden.

6. Verfahren nach einem der vorstehenden Ansprüche, bei dem die Schritte a) bis d) auf einem einheitlichen Druckniveau, insbesondere auf einem Druckniveau von 10 bis 30 bar, beispielsweise von 25 bis 30 bar, durchgeführt werden.

7. Verfahren nach einem der vorstehenden Ansprüche, bei dem das erste Temperaturniveau bei 25 bis 40 °C, das zweite Temperaturniveau bei 5 bis 10 °C und/oder das dritte Temperaturniveau bei -30 bis -40 °C liegt.

8. Verfahren nach einem der Ansprüche 3 bis 7, bei dem der erste Gegenstromwärmetauscher (17) ferner mit Propan oder Propylen als Kältemittel betrieben wird und in dem ersten Gegenstromwärmetauscher (17) ferner Fluid des zweiten Trennabstroms (m) erwärmt wird.

9. Verfahren nach einem der vorstehenden Ansprüche, bei dem zur Bildung des ersten (S) und des zweiten Trennabstroms (m) eine Destillationssäulenanordnung mit einer ersten Destillationssäule (21) und einer zweiten Destillationssäule (23) verwendet wird und der erste Trennabstrom (S) aus Fluid im Sumpf der ersten Destillationssäule (21) und der zweite Trennabstrom (m) aus Fluid am Kopf der zweiten Destillationssäule (23) gebildet wird.

10. Verfahren nach Anspruch 9, bei dem die erste Destillationssäule (21) und die zweite Destillationssäule (23) der Destillationssäulenanordnung als Teil einer Doppelsäule ausgebildet sind, in der die zweite Destillationssäule (23) oberhalb der ersten Destillationssäule (21) angeordnet ist.

11. Verfahren nach Anspruch 9 oder 10, wobei das Fluid des ersten flüssigen Kondensatstroms (c) in die erste Destillationssäule (21) eingespeist wird.

12. Verfahren nach einem der Ansprüche 9 bis 11, bei dem das Fluid des ersten flüssigen Kondensatstroms (c) vor dem Einspeisen in die Destillationssäulenanordnung (21) durch Strippen weiter an Wasserstoff abgereichert wird.

13. Verfahren nach Anspruch 12, bei dem das Fluid des ersten flüssigen Kondensatstroms (c) vor dem Strippen mit Fluid aus dem Sumpf der zweiten Destillationssäule (23) vereinigt wird.

14. Anlage zur Gewinnung von Propylen, die dafür eingerichtet ist, einen Trenneinsatzstrom (G) bereitzustellen, der in einem Dehydrierungsschritt (101) aus Propan gebildetes Propylen sowie Propan, Kohlenwasserstoffe mit zwei Kohlenstoffatomen, Methan und Wasserstoff enthält, wobei eine Trenneinrichtung vorgesehen ist, die dafür eingerichtet ist, aus Fluid des Trenneinsatzstroms (G) auf einem überatmosphärischen Druckniveau einen Propan und Propylen enthaltenden und an Kohlenwasserstoffen mit zwei Kohlenstoffatomen, Methan und Wasserstoff armen ersten Trennabstrom (S) zu bilden, **dadurch gekennzeichnet, dass** die Trenneinrichtung dazu eingerichtet ist,
a) aus Fluid des Trenneinsatzstroms (G) nach Abkühlen von einem ersten auf ein zweites Temperaturniveau einen ersten flüssigen Kondensatstrom (c) und einen ersten gasförmigen Reststrom (b) zu bilden,
b) aus Fluid des ersten gasförmigen Reststroms (b) nach weiterem Abkühlen auf ein drittes Temperaturniveau einen zweiten flüssigen Kondensatstrom (d) und einen zweiten gasförmigen Reststrom (e) zu bilden,
c) aus Fluid des ersten (c) und des zweiten flüssigen Kondensatstroms (d) den ersten (S) und einen Methan und Kohlenwasserstoffe mit zwei Kohlenstoffatomen enthaltenden und an Wasserstoff und Kohlenwasserstoffen mit mehr als drei Kohlenstoffatomen armen zweiten Trennabstrom (m) zu bilden,
d) aus Fluid des zweiten gasförmigen Reststroms (e) einen zumindest Propylen und Propan enthaltenden und an Wasserstoff abgereicherten dritten Trennabstrom (x) zu bilden, und
e) den Trenneinsatzstrom (G) unter Verwendung von Fluid des dritten Trennabstroms (x) zu bilden.

15. Anlage nach Anspruch 14, die zur Durchführung eines Verfahrens nach einem der Ansprüche 1 bis 13 eingerichtet ist.
